# EUROPEAN PATENT SPECIFICATION

(11) **EP 3 888 539 B1**
(45) Date of publication and mention of the grant of the patent: **30.07.2025**
(21) Application number: 19891017.6
(22) Date of filing: 29.11.2019
(51) Int. Cl.: A61B 5/00, G06Q 20/12, G06Q 30/06, G16H 40/00

(54) **METHOD AND SYSTEM FOR SUPPORTING AN ECG EXAMINATION OF A USER**
VERFAHREN UND SYSTEM ZUR UNTERSTÜTZUNG EINER EKG-UNTERSUCHUNG EINES BENUTZERS
MÉTHODE ET SYSTÈME POUR SOUTENIR UN EXAMEN ECG D'UN UTILISATEUR

(30) Priority: 30.11.2018 JP 2018225705
(43) Date of publication of application: 06.10.2021
(73) Proprietor: Toray Industries, Inc., Tokyo 103-8666 (JP)
(72) Inventor: FUJIMORI, Shigeo, Tokyo 103-8666 (JP); ITAGAKI, Ichiro, Tokyo 103-8666 (JP)
(74) Representative: Kador & Partner Part mbB
(86) International application number: PCT/JP2019/046717
(87) International publication number: WO 2020/111218

(56) References cited:
- WO-A1-2015/103620
- WO-A1-2015/168590
- WO-A1-2018/047814
- CA-A1- 3 035 954
- GB-A- 2 355 385
- JP-A- 2018 183 412
- US-A- 5 660 182
- US-A1- 2015 038 823
- US-A1- 2018 067 516
- S. SUAVE LOBODZINSKI ET AL: "New devices for very long-term ECG monitoring", CARDIOLOGY JOURNAL, vol. 19, no. 2, 15 April 2012 (2012-04-15), pages 210 - 214, XP055118224, ISSN: 1897-5593, DOI: 10.5603/CJ.2012.0039

## Description

### FIELD

The present invention relates to an examination support method and an examination support system for supporting an electrocardiogram examination utilizing a wear which measures biological signals of a subject.

### BACKGROUND

An electrocardiogram is widely used in diagnosis of heart diseases such as angina (cardiac infarction) and abnormal cardiac rhythm. Heart diseases do not always appear in electrocardiograms as abnormalities. An abnormality appears in an electrocardiogram upon occurrence of an attack, and thus, an electrocardiogram when an attack does not occur cannot be distinguished from a normal electrocardiogram. Thus, an examination of taking an electrocardiogram for a long period under stress of exercise and in daily life is performed. Typically, a Holter electrocardiogram examination for 24 hours is widely performed as an electrocardiogram examination for a long period. While this electrocardiogram examination is common, missing of a heart disease is pointed out also in the electrocardiogram examination for 24 hours, and a long-term electrocardiogram examination for one week or longer is desired.

As a method for recording an electrocardiogram comfortably and easily under a daily living environment, there is a method for putting a wear on which electrodes and measurement equipment are loaded on a subject and measuring biological signals of the subject. According to this method, the subject can be subjected to measurement for a long period equal to or longer than one week in a comfortable environment. Meanwhile, it is important to prepare a wear fitted for the subject. For example, in a case where a size of the wear is not fitted, electrodes are not fixed at appropriate positions with an appropriate pressure when the subject puts on the wear, which results in failing to obtain stable information with less noise. Further, in the Holter electrocardiogram examination in related art, a subject needs to visit the hospital a number of times (for example, equal to or more than four times) from when a doctor makes a diagnosis until when the subject receives feedback of an examination result, which is heavy load on the subject. Thus, an examination method with less load on the subject is desired.

Meanwhile, as a method for supporting an examination of a patient in a daily living environment, there is a support system of medical equipment disclosed in Japanese Patent Laid-Open No. 2002-109073. In this system, an information provision site determines medical equipment to be provided to a patient at home on the basis of information in accordance with a result of counseling with the patient at home and transmits information regarding the determined medical equipment to a server of a medical equipment support company. The medical equipment support company specifies medical equipment to be provided to the patient at home on the basis of the information received at the server and delivers the specified medical equipment to the patient at home. In this manner, this system provides support of the medical equipment to the patient at home. However, there is no disclosure in Japanese Patent Laid-Open No. 2002-109073 regarding how medical equipment suitable for the patient at home is specifically determined. Particularly, there is no disclosure regarding how an electrocardiogram examination of the patient at home is supported.

Document WO 2015/103620 A1 discloses a method and a system for automatic garment sizing using a video, the garment including ECG equipment and electrodes.

Document US 2018/0067516 A1 discloses a garment with ECG equipment and electrodes, and further includes means for transmitting the recorded ECG data to an external apparatus for analysis.

### PROBLEM TO BE SOLVED

The present invention provides an examination support method and an examination support system for supporting an electrocardiogram examination utilizing a wear which measures biological signals of a subject.

### SUMMARY

The invention is defined by the appended claims 1-12.

### EFFECT ACHIEVED BY THE PRESENT INVENTION

According to the present invention, it is possible to provide an examination support method and an examination support system which can efficiently perform a series of procedure regarding an electrocardiogram examination while reducing load on a subject.

### BRIEF DESCRIPTION OF THE DRAWINGS

FIG. 1 is a block diagram illustrating an entire configuration of an examination support system according to the present embodiment.
FIG. 2 is a front view illustrating an example of a measurement wear according to the present embodiment.
FIG. 3 is a rear view of a front body of the measurement wear according to the present embodiment.
FIG. 4 is an enlarged view of a size adjustment mechanism of the front body of the measurement wear according to the present embodiment.
FIG. 5 is a rear view of the measurement wear according to the present embodiment.
FIG. 6 is a view illustrating an example of an order input screen according to the present embodiment.
FIG. 7 is a view illustrating an example of a wear selection screen according to the present embodiment.
FIG. 8 is a view illustrating an example of the wear selection screen according to the present embodiment.
FIG. 9 is a view illustrating an example of a shipment screen according to the present embodiment.
FIG. 10 is a view illustrating an example where fastened positions are marked according to the present embodiment.
FIG. 11 is a view of a subject seen from a diagonally forward right direction when the subject puts on the measurement wear according to the present embodiment.
FIG. 12 is a rear view of the subject seen from a diagonally backward left direction when the subject puts on the measurement wear according to the present embodiment.
FIG. 13 is a view illustrating an overview of an analysis result according to the present embodiment.
FIG. 14 is a view illustrating part of measured waveforms registered in an analysis report according to the present embodiment.
FIG. 15 is a view illustrating part of compressed waveforms of the analysis report according to the present embodiment.
FIG. 16 is a sequence diagram of the whole operation according to the present embodiment.

### DETAILED DESCRIPTION

Outline of the present embodiment will be described first. A subject visits a medical institution, and a doctor diagnoses the subject. In a case where the doctor judges that the subject is suspected to have a heart disease such as angina (cardiac infarction) and abnormal cardiac rhythm, the doctor determines to perform a Holter electrocardiogram examination (hereinafter, an electrocardiogram examination) according to the present embodiment and prescribes an electrocardiogram examination. A laboratory technician measures a body size of the subject as information necessary for determining the size of the measurement wear (wear) to be put on and obtains size information on the subject including an under bust size. Health personnel (a doctor, a laboratory technician or other health personnel to whom the doctor provides directions) of the medical institution orders a wear to be put on the subject to a medical equipment sales agency using an information processing apparatus. The health personnel also notifies the size information on the subject upon ordering. The agency determines a wear of a size appropriate for the subject on the basis of the size information on the subject and sends the determined wear of the size to the subject along with an instruction manual. The subject who has received the wear puts on the wear along the instruction manual and measures biological signals (electrocardiogram signals) during a measurement period (for example, one week). After the measurement period, the subject sends back the wear which the subject has put on to the medical equipment sales agency. The medical equipment sales agency reads out measurement data from measurement equipment (cardiography equipment) loaded on the wear and requests analysis of the readout measurement data to an electrocardiographic analysis institution. The electrocardiographic analysis institution analyzes the measurement data and creates an analysis report. The medical institution receives the analysis report from the electrocardiographic analysis institution, and the doctor diagnoses a state of the subject using the analysis report.

The present embodiment provides an examination support method and an examination support system for efficiently performing a series of procedure regarding an electrocardiogram examination as described above while reducing load on a subject.

An embodiment of the present invention will be described in detail below with reference to the drawings.

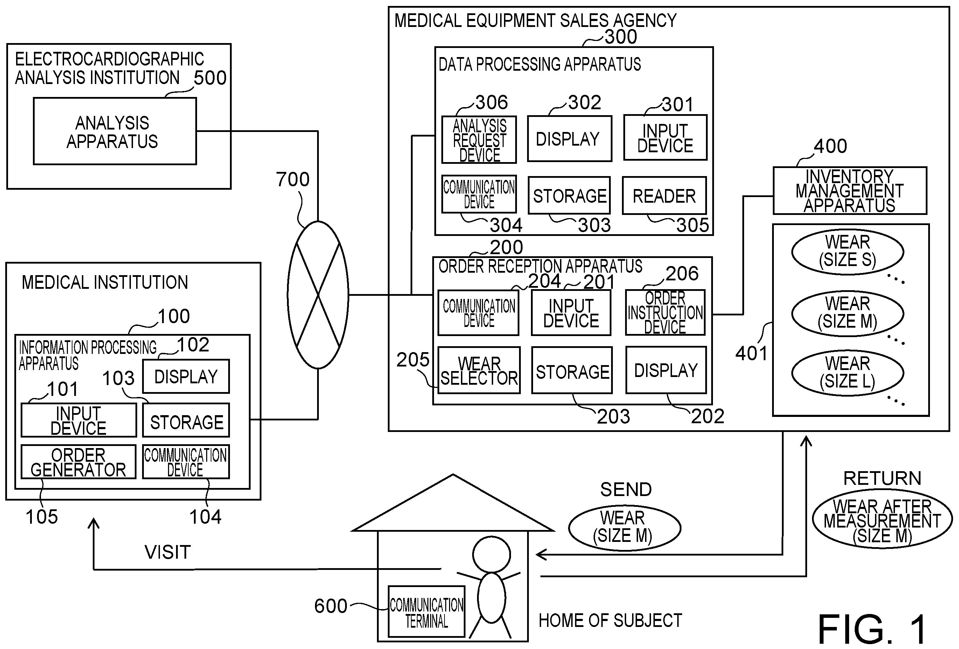

FIG. 1 is a block diagram illustrating an entire configuration of an examination support system according to the present embodiment. The examination support system in FIG. 1 includes an information processing apparatus 100 disposed at a medical institution, an order reception apparatus 200, a data processing apparatus 300 and an inventory management apparatus 400 disposed at a medical equipment sales agency, an analysis apparatus 500 disposed at an analysis center, a communication terminal 600 of the subject and a communication network 700.

The medical institution is an institution which provides medical service including an electrocardiogram examination, and the like, to the subject. As an example, the medical institution is a hospital, and the subject is a patient who is to undergo an electrocardiogram examination. A doctor diagnoses the subject, and in a case where the doctor judges that the subject is suspected to have a heart disease such as abnormal cardiac rhythm, the doctor determines to perform an electrocardiogram examination and prescribes the electrocardiogram examination.

The electrocardiogram examination uses a measurement wear (hereinafter, a wear) on which measurement equipment (cardiography equipment) which measures action potential or an action current of cardiac muscle associated with beat of a heart as a biological signal of the subject is loaded. The subject puts on the wear during a measurement period. The measurement period is determined by the doctor and is approximately from one to two weeks as an example. Measurement data recorded by the cardiography equipment is called an electrocardiogram. To order a wear to be put on the subject, a laboratory technician measures the body size of the subject as information necessary for determining the size of the wear. However, the measurement may be performed by the doctor or other health personnel to whom the doctor provides directions. Further, measurement may be performed using a measurement apparatus which automatically measures the body size, or the laboratory technician, or the like, may manually measure the body size using a measuring tape.

Measurement items of the body size include a body height, a chest measurement, under bust, a shoulder width, a sitting height, a body weight, and the like. The measurement items do not have to include all of these and may include other items. However, it is assumed in the present embodiment that the measurement items include at least under bust.

Here, a wear to be used in the present embodiment will be described.

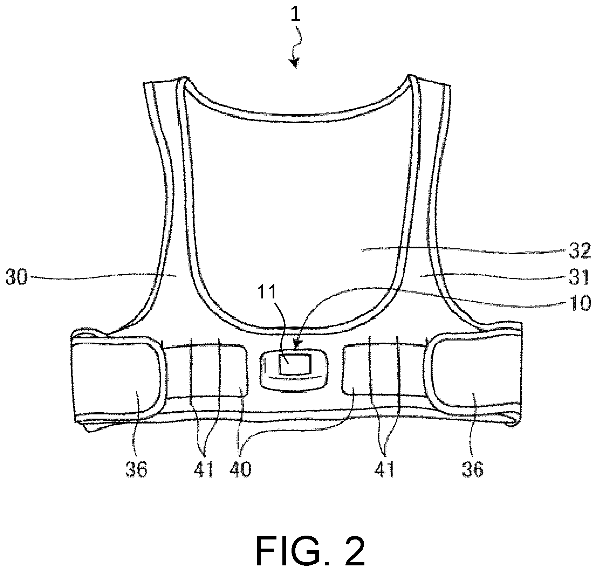

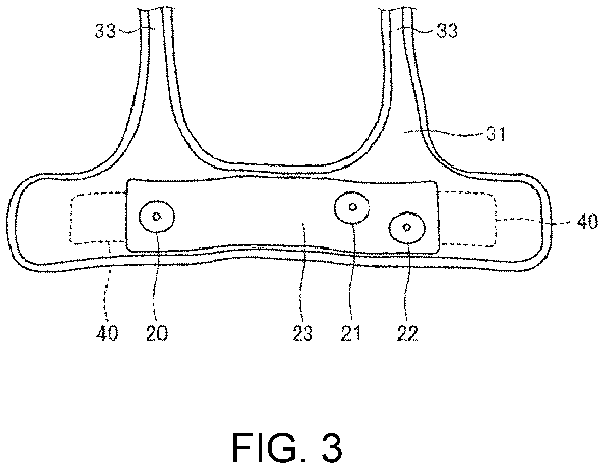

FIG. 2 is a front view of a wear 1. FIG. 3 is a view of a reverse side (side in contact with the skin) of a front body of the wear 1. The wear 1 includes cardiography equipment 10 which is measurement equipment, electrodes 20, 21 and 22 (FIG. 3), and a wear body portion 30. Medical equipment in the present embodiment includes the cardiography equipment 10 and the electrodes 20, 21 and 22.

The wear body portion 30 includes a front body 31 and a rear body 32. The front body 31 is connected to the rear body 32 with two shoulder belts (shoulder straps) 33 illustrated in FIG. 3, and the front body 31 is separated from the rear body 32 at both sides (side portions). The cardiography equipment 10 is loaded at the center of a torso portion of the front body 31. On the rear side of the torso portion of the front body 31 on which the cardiography equipment 10 is loaded, electrodes 20, 21 and 22 (FIG. 3) which make contact with the skin of the subject are attached.

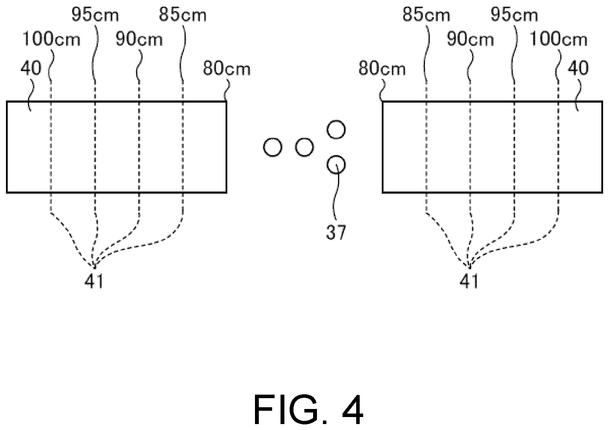

These electrodes 20, 21 and 22 are arranged on the basis of CC5 which is one of induction methods of the Holter electrocardiogram examination. The cardiography equipment 10 and the electrodes 20, 21 and 22 are located around the under bust of the wearer when the wearer puts on the wear. The electrode 20 becomes a positive electrode, the electrode 21 becomes a negative electrode, and the electrode 22 becomes an earth electrode. The electrodes 20, 21 and 22 are constituted with conductive fiber as an example. However, the electrodes 20, 21 and 22 are not limited to conductive fiber, and may be adhesive films including a conductive material. A size and a shape of the electrodes 20, 21 and 22 are not particularly limited as long as the electrodes can detect biological signals. While not illustrated, the respective electrodes 20, 21 and 22 are connected to a connector 37 (see FIG. 4) of the cardiography equipment 10 with a lead line. These wiring portions are covered with an electronic insulating member 23.

The electrodes 20, 21 and 22 may be detachable from the front body 31. Further, the cardiography equipment 10 may be detachable from the wear body portion 30 via the connector 37 (see FIG. 4). By detaching at least the cardiography equipment 10 among the cardiography equipment 10 and the electrodes 20 to 22 from the wear body portion 30, it is possible to wash the wear. As the connector 37, a socket, or the like, which is typically used in connection of cords can be used as an example. A plurality of metal dot buttons which can fix the cardiography equipment 10 at the wear body portion 30 at the same time may be used.

The cardiography equipment 10 includes a chargeable battery and can record electrocardiogram data (measurement data) for one or two weeks, or more weeks without being additionally charged, in a state where the battery is fully charged. The cardiography equipment 10 includes a storage which can store all data measured during one or two weeks or more weeks. The cardiography equipment 10 includes a communication device which performs communication with apparatuses such as a computer wiredly or wirelessly. The communication device has a function of connecting with the apparatus wiredly or wirelessly and transmitting the measurement data stored in the storage to the apparatus. Further, the cardiography equipment 10 includes a display 11 which displays data. The cardiography equipment 10 can display an operation state of the cardiography equipment 10 at the display 11 during operation. The subject can grasp whether or not the cardiography equipment 10 normally operates, that is, whether or not measurement is normally performed by confirming the display 11 during the measurement period.

At the torso portion of the front body 31, a hook and loop fastener B side (loop side) 40 is sewed so that a flat rubber embedded in the rear body 32 can be fixed with an equal extension degree in accordance with the size of the under bust of the wearer. Further, side tabs 36 are attached at both ends of the torso portion of the rear body 32. The front body 31 is connected to the rear body 32 at both sides (side portions) by a hook and loop fastener A side (hook side) provided on a rear side of the side tabs 36 being fixed with the hook and loop fastener B side 40 of the front body 31. When the hook and loop fastener A side is fixed, the side tabs 36 are located around the under bust. The hook and loop fastener A side and the hook and loop fastener B side 40 function as a size adjustment mechanism for adjusting a pressure when the subject puts on the wear. While the present embodiment includes two right and left size adjustment mechanisms, the number of size adjustment mechanisms may be one or three or more.

A plurality of stitches 41 are provided on the hook and loop fastener B side 40 at intervals to make it easier to understand fastened positions of the side tabs 36. The stitches 41 correspond to scales indicating candidates for fastened positions of the side tabs 36 (adjustment positions for positioning). However, the side tabs 36 do not have to be fastened at the positions of scales and may be fastened at positions between scales. Further, one stitch 41 may be provided instead of a plurality of stitches 41 being provided. The stitches 41 are constituted with colored yarn different from color of the hook and loop fastener B side 40 as an example.

An interval of scales is equal to or greater than 1 cm and equal to or less than 8 cm at each of the right and left size adjustment mechanisms as an example. In a case where there is one size adjustment mechanism, the interval of scales is equal to or greater than 2 cm and equal to or less than 16 cm as an example.

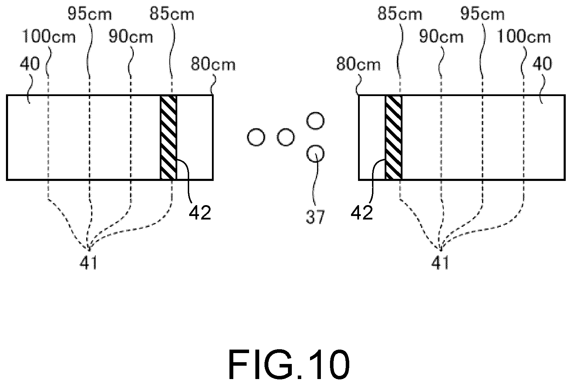

FIG. 4 is an enlarged view of the hook and loop fastener (B side) of the front body 31. FIG. 4 illustrates an example of a wear of M size which is to be applied to a size of the under bust of the subject from 80 cm to 100 cm. The subject fastens tips of the side tabs 36 at appropriate positions (fastened positions) in accordance with the size of the under bust. For example, in a case where the size of the under bust of the subject is 90 cm, the subject fastens the tips of the right and left side tabs 36 so as to be respectively aligned with positions of the second stitches from the connector 37 of the cardiography equipment 10. Further, in a case where the size of the under bust is 87 cm, the subject fastens the tips of the side tabs 36 so as to be aligned with positions moved from positions of the first stitches to the second stitches by 1 cm. In the present embodiment, the wear is provided to the subject in a state where the fastened positions are marked (see FIG. 10 which will be described later) so as to prevent the subject from fastening the tips of the side tabs 36 at wrong positions or to prevent the subject from forgetting the fastened positions.

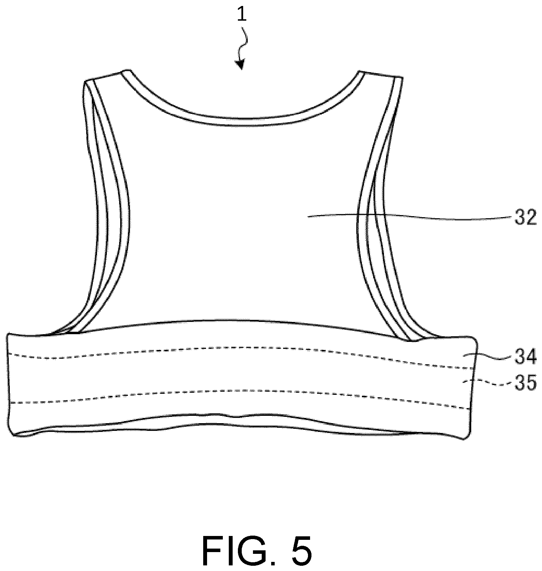

FIG. 5 is a rear view of the wear 1. A flat rubber is embedded into the torso portion 34 of the rear body 32 as an elastic body 35. As an example, by setting a length of the elastic body 35 at equal to or higher than 30% and equal to or less than 60% of the under bust of the subject, it is possible to acquire biological signals while the electrodes 20, 21 and 22 supported at the wear body portion 30 make contact with the skin of the subject with an appropriate pressure, that is, without the subject feeling strong compression by putting on the wear. Further, the elastic body 35 is approximately from 25 mm to 50 mm wide as an example. The elastic body 35 requires force equal to or greater than 3N and equal to or less than 9N to extend in a long axis direction by 30% as an example. Further, the elastic body 35 requires force equal to or greater than 2N and equal to or less than 6N to extend in the long axis direction by 20% as an example.

While FIG. 4 illustrates an example of the wear of the M size which is to be applied to the size of the under bust of 80 cm to 100 cm, a plurality of sizes are prepared in accordance with ranges of the size of the under bust. As an example, an S size is prepared for the size of the under bust of 60 cm to 80 cm, and an L size is prepared for 100 cm to 120 cm. A size of the wear body portion (a length (height) in a longitudinal direction and a length (width) in a lateral direction on paper in FIG. 2) is different depending on a difference in a size of the wear. Further, in each size, the hook and loop fastener (B side) and respective stitches on the hook and loop fastener (B side) are arranged so as to be able to fix the side tabs at appropriate positions in accordance with the size of the under bust of the wearer. Numerical values described here are merely an example. For example, the size of the wear may be prepared for each size range such as 10 cm in place of 20 cm. Further, the ranges of the under bust may overlap between sizes. For example, the M size may be prepared for 80 cm to 100 cm, and the L size may be prepared for 95 cm to 115 cm. Further, other sizes such as an SS size or an LL size may be provided. Still further, only two sizes may be provided for the wear.

The doctor, the laboratory technician or other health personnel orders a wear to the medical equipment sales agency with which they have partnership in advance using the information processing apparatus 100. Hereinafter, a person who operates the information processing apparatus 100 will be referred to as an operator. The operator inputs information necessary for ordering the wear to the information processing apparatus 100 and transmits order information on the wear to the order reception apparatus 200 of the medical equipment sales agency using the information processing apparatus 100. Note that in a case where a plurality of doctors and laboratory technicians, and the like, operate the information processing apparatus 100, the information processing apparatuses operated by the respective persons may be the same apparatus or may be different apparatuses having similar functions.

As illustrated in FIG. 1, the information processing apparatus 100 includes an input device 101, a display 102, a storage 103, a communication device 104 and an order generator 105. A hardware configuration of the information processing apparatus 100 is similar to that of a typical computer including a processor such as a CPU, a storage apparatus, a display apparatus, an input apparatus, a communication interface, and the like.

The input device 101 is an apparatus for inputting data, and, for example, is a mouse, a keyboard, a touch panel, or the like. The input device 101 may be a speech input device such as a microphone which performs input using speech. Examples of data to be input can include instruction information by the operator, information on the subject, or the like.

The display 102 is a display apparatus which displays data, and is, for example, a liquid crystal display apparatus, an organic EL display apparatus, a CRT display apparatus, or the like.

The storage 103 is an apparatus which stores data. The storage 103 stores data input from the input device 101. Further, the storage 103 stores various kinds of application (such as an operating system (OS) and order application) necessary for implementing operation of the present embodiment. Further, the storage 103 may store information on the subject as an electronic medical record. Further, the storage 103 may store data generated by the order generator 105 which will be described later, and data received at the communication device 104. Specific examples of the storage 103 can include a memory apparatus, a hard disk, an SSD, or the like. The storage 103 may be connected from outside so as to be detachable via a connection interface (such as a USB port and a memory slot) provided at the information processing apparatus 100.

The communication device 104 is connected to the communication network 700 wiredly or wirelessly and performs communication with other apparatuses in accordance with communication protocol. Specific examples of other apparatuses can include the order reception apparatus 200, the data processing apparatus 300, the analysis apparatus 500 and the communication terminal 600.

The order generator 105 generates an order input screen for ordering a wear and causes the order input screen to be displayed at the display 102. The order generator 105 is implemented by a processor such as a CPU being caused to execute the order application. The operator inputs information necessary for ordering on the order input screen via the input device 101.

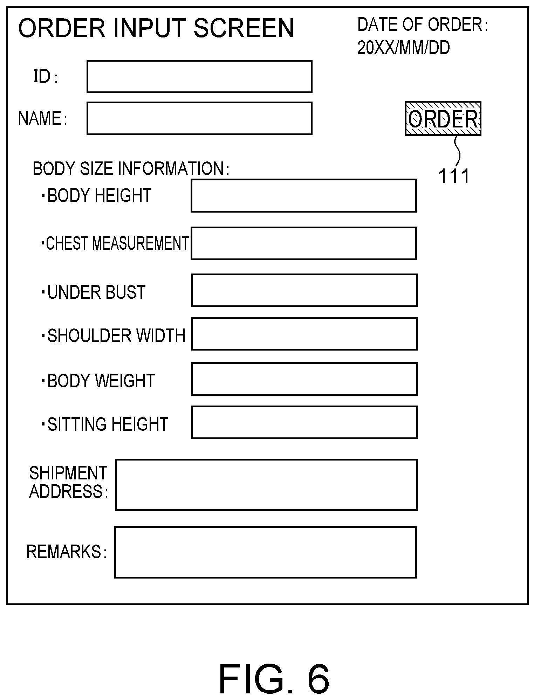

FIG. 6 illustrates an example of the order input screen. The order input screen includes a plurality of input items including an ID of the subject, name of the subject, size information on the subject, shipping address of the wear (for example, a dwelling place or address of the subject) and remarks. Today's date is displayed as date of order at an upper right portion of the screen. Arbitrary information is input in the remarks. For example, date and time at which the subject desires to receive the wear may be designated and input. Information (such as name) of the medical equipment sales agency may be displayed on this order input screen. Further, email address information on the order reception apparatus 200 may be displayed. A transmission instruction is provided to the order application by the operator entering data at respective input items and clicking an order button 111.

The order application generates order information on the wear upon receipt of the transmission instruction from the operator. The order information includes various kinds of information input on the order input screen as an example. The order generator 105 outputs the generated order information to the communication device 104. The communication device 104 transmits the order information to the order reception apparatus 200 via the communication network 700. The order information is transmitted by email as an example. In this case, email address information on the order reception apparatus 200 which is a transmission destination of the order information may be set in advance in the order application or may be input by the operator on the order input screen.

A transmission method of the order information is not limited to email. For example, a web server for receiving orders may be provided on the order reception apparatus 200 side, and the order generator 105 may access an URL of the web server. In this case, the order information is transmitted to the order reception apparatus 200 by the operator inputting information necessary for ordering from a screen which is provided by the web server and which is displayed at the display 102 of the information processing apparatus 100. Alternatively, the order generator 105 may transmit the order information via fax machine connected to the information processing apparatus 100 via a network. In this case, the fax machine may transmit the order information in a form of an electronic file to the order reception apparatus 200. Alternatively, the order generator 105 may transmit the order information to a printer connected to the information processing apparatus 100 via a network and may cause the printer to print the order information. In this case, the operator may send a sheet which describes the order information to the medical equipment sales agency by mail or by courier or by fax.

A function of inputting an order on a screen of an electronic medical record of the subject may be provided. In this case, the screen of the electronic medical record can be used as the order input screen. In this event, a function of inputting an order instruction such as an order button or order menu is added on the screen of the electronic medical record. In a case where the electronic medical record does not include part of items required for ordering the wear, it is only necessary to add the items to the electronic medical record.

The medical equipment sales agency is a medical equipment sales company. The medical equipment sales company accepts an order of the wear from the medical institution (information processing apparatus 100). The medical equipment sales company determines a size of the wear to be provided to the subject and fastened positions of the side tabs using the order reception apparatus 200. The medical equipment sales agency prepares the wear of the determined size, marks the determined fastened positions of the side tabs and sends the wear to the subject.

In more detail, the order reception apparatus 200 includes an input device 201, a display 202, a storage 203, a communication device 204, a wear selector 205, and a shipment instruction device 206. A hardware configuration of the order reception apparatus 200 is similar to that of a typical computer including a processor such as a CPU, a storage apparatus, a display apparatus, an input apparatus, a communication interface, and the like. The order reception apparatus 200 is operated by a staff of the medical equipment sales agency.

The input device 201, the display 202, the storage 203, and the communication device 204 have functions similar to those of the input device, the display, the storage and the communication device of the information processing apparatus 100 except content or a type of data or application to be dealt with.

The communication device 204 passes the order information received from the information processing apparatus 100 to order reception application. The order reception application holds the order information which has received so far. The order reception application displays a list of the order information. The staff selects unprocessed order information and displays content of the selected order information on a screen. The staff activates wear selection application for determining a size of the wear and the fastened positions of the side tabs appropriate for the subject. The wear selection application is executed by a processor such as a CPU. By this means, functions of the wear selector 205 are implemented. The wear selector 205 displays a screen of the wear selection application (wear selection screen) at the display 202.

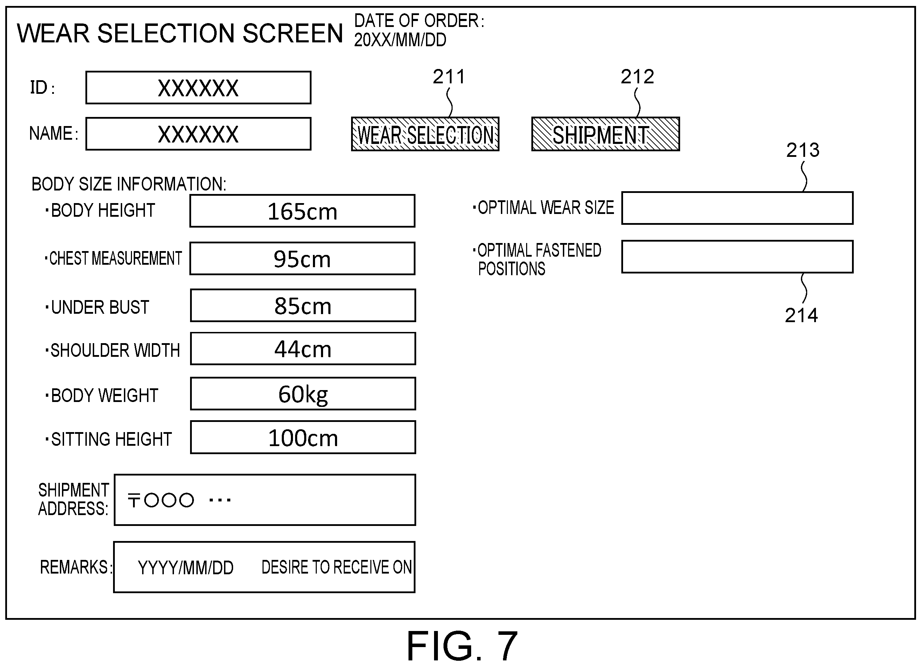

FIG. 7 illustrates an example of the wear selection screen. Content of the selected order information is displayed on the wear selection screen. Further, a wear selection button 211 to be used by the staff to give an instruction of wear selection and calculation of the fastened positions and a shipment button 212 for shipment of the wear are disposed. Further, the wear selection screen includes a field 213 for storing information on an optimal wear size calculated for the subject, and a field 214 for storing information on optimal fastened positions determined for the subject. At this point, no value is stored in the fields 213 and 214.

When the staff clicks the wear selection button 211, a wear selection instruction is provided to the wear selection application. The wear selection application selects a size of the wear optimal for the subject and calculates optimal fastened positions of the side tabs on the basis of the size information on the subject.

In the present embodiment, the size of the wear and the fastened positions are determined on the basis of the under bust size of the subject. As an example, the wear size includes S, M and L. A plurality of fastened positions are set in accordance with each size. For example, in an example of the size M illustrated in FIG. 4, the fastened positions are set at 1 cm intervals for the under bust from 80 cm to 100 cm. A setting example of the fastened positions will be described below. P_{M}1, P_{M}2, ..., P_{M}21 are identifiers of the respective fastened positions.
80 cm: position of the first stitch (P_{M}1)
81 cm: position moved from the position of the first stitch to the second stitch by 5 mm (P_{M}2)
82 cm: position moved from the position of the first stitch to the second stitch by 1 cm (P_{M}3)
83 cm: position moved from the position of the first stitch to the second stitch by 1.5 cm (P_{M}4)
84 cm: position moved from the position of the first stitch to the second stitch by 2 cm (P_{M}5)
85 cm: position of the second stitch (P_{M}6)
86 cm: position moved from the position of the second stitch to the third stitch by 5 mm (P_{M}7)
87 cm: position moved from the position of the second stitch to the third stitch by 1 cm (P_{M}8)
88 cm: position moved from the position of the second stitch to the third stitch by 1.5 cm (P_{M}9) ...
99 cm: position moved from a position of the third stitch to the fourth stitch by 2 cm (P_{M}20)
100 cm: position of the fourth stitch (P_{M}21)

Also in a case of the size S, a plurality of fastened positions P_{S}1, P_{S}2, ..., P_{S}21 are set at 1 cm intervals in a range of the under bust from 60 cm to 80 cm in a similar manner. Also in a case of the size L, a plurality of fastened positions P_{L}1, P_{L}2, ..., P_{L}21 are set at 1 cm intervals in a range of the under bust from 100 cm to 120 cm. While an example where the fastened positions are set at 1 cm intervals has been described here, the interval may be 0.5 cm, or may be other values. It is only necessary to determine the interval in accordance with granularity of a measurement size of the under bust.

The wear selection application holds correspondence information in which sizes of the wear are associated with the fastened positions of the side tabs for each under bust size as an example. The correspondence information may have a table form or may be embedded in the wear selection application as a program code.

The wear selection application determines the size of the wear and the fastened positions of the side tabs which are optimal for the subject on the basis of the under bust size of the subject and the above-described correspondence information. For example, in a case where the under bust size of the subject is 85 cm, the wear selection application determines that the wear size optimal for the subject is M, and the fastened positions of the side tabs optimal for the subject are P_{M}6. The wear selection application stores values indicating the determined wear size and the determined fastened positions of the side tabs in the fields 213 and 214 on the wear selection screen.

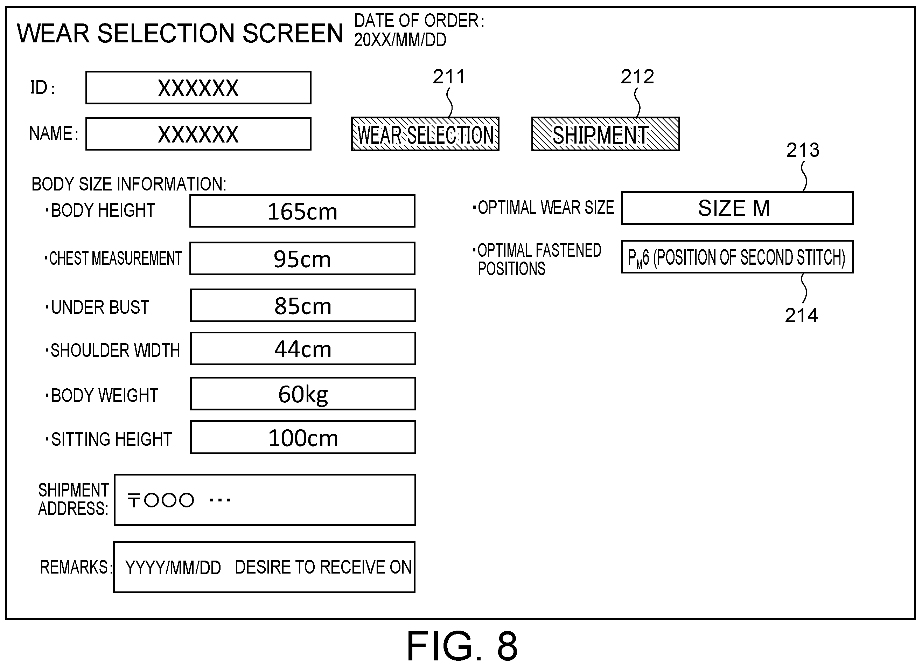

FIG. 8 illustrates an example where the values indicating the size of the wear and the fastened positions determined by the wear selection application are stored in the fields 213 and 214 on the wear selection screen. The wear size M and the fastened positions P_{M}6 of the side tabs are determined for the subject.

While the size of the wear and the fastened positions of the side tabs have been determined on the basis of the under bust size here, a determination method is not limited to this. For example, the size of the wear and the fastened positions of the side tabs may be determined further using at least one of a body height, a chest measurement, a sitting height and a body weight of the subject. For example, the size of the wear and the fastened positions of the side tabs may be calculated on the basis of the under bust and the chest measurement. As described above, the size is not limited to S, M and L, and more or less sizes may be provided.

Further, the size of the wear and the fastened positions of the side tabs may be determined by the staff. In this case, the staff determines the size of the wear and the fastened positions of the side tabs from the above-described correspondence information on the basis of the under bust size of the subject. The staff inputs values of the determined wear size and fastened positions in the fields 213 and 214.

After the values of the wear size and the fastened positions are stored in the fields 213 and 214, a shipment screen for performing shipment procedure is displayed by the staff clicking the shipment button 212.

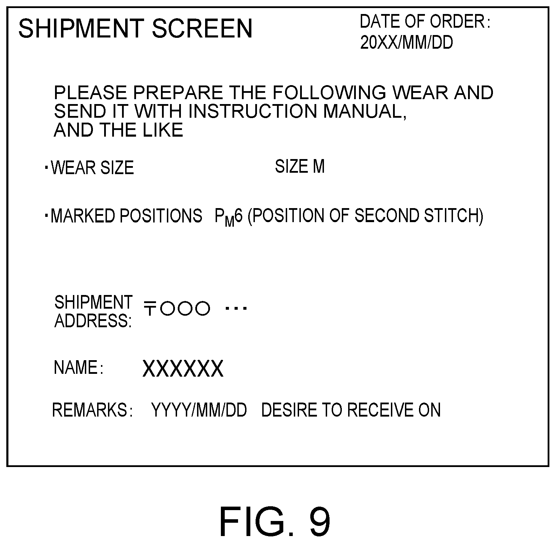

FIG. 9 illustrates an example of the shipment screen. The staff prepares the wear and marks the fastened positions with the content displayed on the shipment screen and sends an electrocardiogram measurement set including the wear on which the marks are provided, an instruction manual, and the like, to the subject, specifically, as follows.

After the staff confirms the wear size and the fastened positions of the side tabs optimal for the subject, the staff operates the inventory management apparatus 400 to confirm whether or not the wear of the size is left in stock. The inventory management apparatus 400 is an apparatus for managing inventory of the wears which are stored for each size within a warehouse 401. The inventory management apparatus 400 is a computer including an input apparatus, a display apparatus, a processor such as a CPU and a communication apparatus. The staff picks up the wear of the size of the subject from the warehouse 401 after confirming whether or not the wear is left in stock and marks the fastened positions for the subject on the wear. The fastened positions may be marked with a felt pen, with yarn of specific color, with an arrow mark or using other methods.

FIG. 10 illustrates an example where the positions of the second stitches (P_{M}6) on the wear of the size M illustrated in FIG. 4 are marked. Marks 42 are provided at the second stitches.

The fastened positions may be marked with machine which is prepared for marking as well as being marked by the staff. For example, data indicating the fastened positions are input to the machine, and the wear on which the marks are to be provided is placed at the machine. The machine automatically provides marks on the wear by the staff giving a marking instruction to the machine. The data indicating the fastened positions may be automatically input to the machine by the machine being made to coordinate with the order reception apparatus 200 or the inventory management apparatus 400.

The wears on which the respective fastened positions (P_{S}1 to P_{S}21 in a case of the S size, P_{M}1 to P_{M}21 in a case of the M size, and P_{L}1 to P_{L}21 in a case of the L size) are marked may be prepared in advance and stored in the warehouse 401. In this case, the staff only requires to pick up the wear of the size of the subject, on which the fastened positions determined for the subject are marked from the warehouse 401.

While the order reception apparatus 200 and the inventory management apparatus 400 are separately provided in FIG. 1, the order reception apparatus 200 and the inventory management apparatus 400 may be integrated by the functions of the inventory management apparatus 400 being added to the order reception apparatus 200. Further, the inventory management apparatus 400 does not have to be provided.

The staff packages the electrocardiogram measurement set including the wear on which the marks are provided, the instruction manual, and the like, and sends the set to the subject at shipment address of the subject. The set may be sent by courier or by mail or other ways. Note that an invoice to be pasted on the package may be automatically printed by the shipment application, the wear selection application, or the like, outputting shipment information on the subject to a printer at a predetermined timing (for example, at a timing at which a shipment instruction is given) or may be created through operation by the operator.

While the staff picks up the wear from the warehouse and packages and sends the wear here, all or part of these series of operation can be automatically performed by utilizing a drone or an autonomous driving car.

For example, an automated conveyance apparatus (which may be a mobile robot, a belt conveyer or other types of apparatuses) which coordinates with the order reception apparatus 200 or the inventory management apparatus 400 may be provided in the warehouse 401 and may be caused to convey wears in the warehouse 401. For example, in a case where the shipment button 212 is clicked on the wear selection screen, a conveyance instruction is transmitted from the order reception apparatus 200 to the automated conveyance apparatus directly or via the inventory management apparatus 400 at the same time or before or after the shipment screen is displayed. The automated conveyance apparatus automatically picks up the wear of the size designated in the conveyance instruction from a shelf on which wears are stored for each size and places the wear to a predetermined location. The staff picks up the wear from the predetermined location.

Further, in a case where wears with marks are stored, the automated conveyance apparatus automatically passes the conveyed wear to an automated packaging apparatus. The automated packaging apparatus packages the wear and creates and pastes an invoice. The automated packaging apparatus passes the package to an automated transfer apparatus (such as a drone and an autonomous driving car) and the automated transfer apparatus automatically sends the package to the shipment address of the subject. The shipment address may be transmitted from the inventory management apparatus 400 or the order reception apparatus 200 to the automated transfer apparatus or may be read by the automated transfer apparatus from the invoice. There are various possible methods for sending the wear to the subject after the wear size and the fastened positions optimal for the subject are determined in this manner, and any method may be used.

The subject receives the electrocardiogram measurement set sent from the medical equipment sales agency. The subject puts on the wear in accordance with the instruction manual included in the package and explanation received from the health personnel (such as the doctor and the laboratory technician) of the medical institution in advance and records an electrocardiogram. For example, the subject puts on the wear directly on the skin of the upper body and aligns the tips of the side tabs with the marked positions to thereby fix the wear on the body.

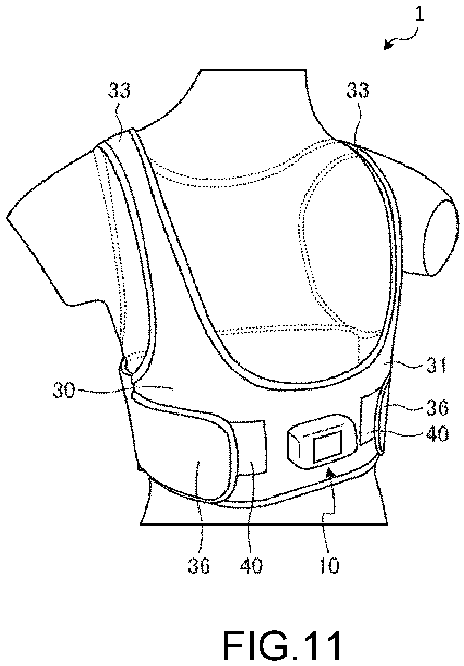

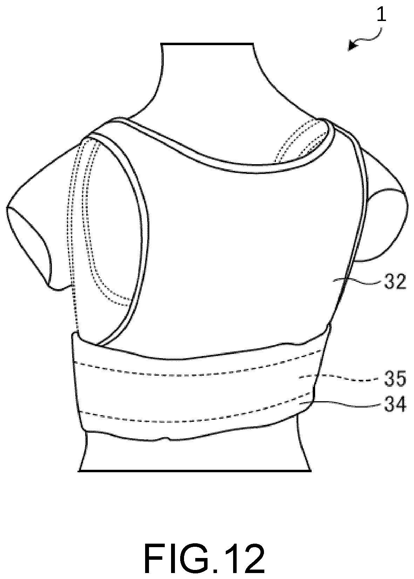

FIG. 11 is a view of the subject seen from a diagonally forward right direction when the subject puts on the wear 1. FIG. 12 is a rear view of the subject seen from a diagonally backward left direction when the subject puts on the wear 1. Misalignment of the wear body portion 30 is prevented because the front body 31 is connected to the rear body 32 with the shoulder belt 33. The wear of the size appropriate for the subject is provided, and the subject fixes the tips of the side tabs at appropriate fastened positions, so that the electrodes 20, 21 and 22 (FIG. 3) make contact with the skin of the subject with an appropriate pressure. The subject therefore does not feel strong compression by putting on the wear. This enables the subject to record an electrocardiogram in a comfortable environment and enables biological signals of the subject to be acquired at an appropriate level which allows correct electrocardiographic analysis. Further, marking of the fastened positions allows the subject to easily understand the positions where the side tabs 36 are to be fastened.

The subject activates the cardiography equipment by powering on the cardiography equipment before or after putting on the wear. By this means, measurement is started. The measurement is performed for a measurement period (for example, one week) determined on the basis of the diagnosis by the doctor. The subject keeps the wear on and intermittently performs measurement except a limited period while the subject changes his/her clothes, takes a bath, or the like. In a case where the cardiography equipment and the electrodes are detachable from the wear, the subject may detach the cardiography equipment and the electrodes from the wear and may wash or clean a wear portion.

The subject has the communication terminal 600. The communication terminal 600 is a terminal such as a smartphone, a mobile phone, a tablet terminal, a notebook personal computer (PC) and a desktop PC. The subject may contact the health personnel (such as the doctor and the laboratory technician) of the medical institution or the staff of the medical equipment sales agency using the communication terminal 600 by email, by phone, or the like.

The subject returns the wear which the subject has put on to the medical equipment sales agency after the measurement period. The wear may be returned using a method designated by the medical equipment sales agency in advance such as by mail or by courier. In a similar manner to a case where the wear is sent to the subject, the wear may be returned using a drone, an autonomous driving car, or the like. The cardiography equipment may have a built-in timer, sound source and speaker, and may notify end of the measurement period to the subject with the timer. In place of or in addition to the sound source and the speaker, the cardiography equipment may include a vibrator and may notify end of the measurement period to the subject by driving the vibrator. The subject makes settings of the timer upon start of measurement as an example. Further, information on the measurement period may be notified from the information processing apparatus 100 to the order reception apparatus 200, and instructions which describe the measurement period may be included in the electrocardiogram measurement set sent to the subject. In a case where a duration of the measurement period is determined in advance, operation of notifying the information on the measurement period from the information processing apparatus 100 to the order reception apparatus 200 is not required.

The data processing apparatus 300 of the medical equipment sales agency includes an input device 301, a display 302, a storage 303, a communication device 304, a reader 305, and an analysis request device 306. A hardware configuration of the data processing apparatus 300 is similar to that of a typical computer including a processor such as a CPU, a storage apparatus, a display apparatus, an input apparatus, a communication interface, and the like. The data processing apparatus 300 may be the same hardware apparatus as the order reception apparatus 200 or the inventory management apparatus 400 or may be a hardware apparatus different from the order reception apparatus 200 or the inventory management apparatus 400. The data processing apparatus 300 is operated by the staff of the medical equipment sales agency.

The input device 301, the display 302, the storage 303, and the communication device 304 have functions similar to those of the input device, the display, the storage and the communication device of the information processing apparatus 100 or the order reception apparatus 200 except content or a type of data or application to be dealt with.

The staff connects the cardiography equipment loaded on the wear returned from the subject to the reader 305 wiredly or wirelessly and causes the reader 305 to read out measurement data (electrocardiogram data) stored in the cardiography equipment. The reader 305 writes the read measurement data in the storage 303 as an electrocardiographic file. Further, the reader 305 may write the read data in a computer readable recording medium. Examples of the recording medium can include a CD-R, a USB memory, an SSD, a memory card, and the like.

There are various configurations where the reader 305 reads out measurement data from the cardiography equipment. For example, the measurement data may be read out from the cardiography equipment through wireless local area network (LAN) communication, Bluetooth, near field communication, or the like. Further, the reader 305 may be connected to the cardiography equipment wiredly using a serial cable, or the like, and the measurement data may be transmitted from the cardiography equipment to the reader 305 through serial transfer. Further, a dedicated data transfer scheme may be defined, and the measurement data may be transferred in accordance with this scheme. The measurement data may be transferred using a method other than the methods described here.

The analysis request device 306 is implemented by a processor such as a CPU being caused to execute analysis request application. The staff selects the measurement data (electrocardiographic file) for which an analysis request is to be made and gives an instruction to generate and transmit analysis request information via a user interface screen provided by the analysis request application.

The analysis request device 306 generates analysis request information for requesting analysis of the electrocardiogram for the measurement data selected by the staff. The analysis request information includes the measurement data for which an analysis request is to be made, information on the subject, and the like. The information on the subject may include name and an ID of the subject or may include only an ID. The analysis request device 306 outputs the generated analysis request information to the communication device 304.

The communication device 304 transmits the received analysis request information to an analysis apparatus 500 of the electrocardiographic analysis institution via the communication network 700. Any method may be used to transmit the analysis request information to the analysis apparatus 500. The analysis request information may be transmitted by e-mail or may be transmitted using a file exchange system or may be uploaded to a dedicated web server prepared at the electrocardiographic analysis institution. Further, the analysis request information to be transmitted may be encrypted.

While the analysis request information is transmitted via the communication network 700 here, a recording medium in which the measurement data is stored and an analysis request form may be transmitted to the electrocardiographic analysis institution by mail, by courier, or the like. Further, the measurement data and the analysis request form may be transmitted to the electrocardiographic analysis institution by fax. The measurement data and the analysis request form may be provided to the electrocardiographic analysis institution using other methods.

The analysis apparatus 500 provided at the electrocardiographic analysis institution receives the analysis request information from the data processing apparatus 300. A hardware configuration of the analysis apparatus 500 is similar to that of a typical computer including a processor such as CPU, a storage apparatus, a display apparatus, an input apparatus, a communication interface, and the like. The analysis apparatus 500 is operated by a staff of the electrocardiographic analysis institution.

The staff operates the analysis apparatus 500 to display the analysis request information received from the data processing apparatus 300 on a screen. The analysis apparatus 500 is equipped with electrocardiographic analysis application. The staff activates the electrocardiographic analysis application, causes the electrocardiographic analysis application to load the measurement data included in the analysis request information and execute analysis processing. The analysis apparatus 500 displays an analysis result on a screen.

The analysis apparatus 500 creates an analysis report on the basis of the analysis result. The analysis report includes information on the measurement period of the subject, information on the subject, the analysis result of the electrocardiogram, and the like, as an example.

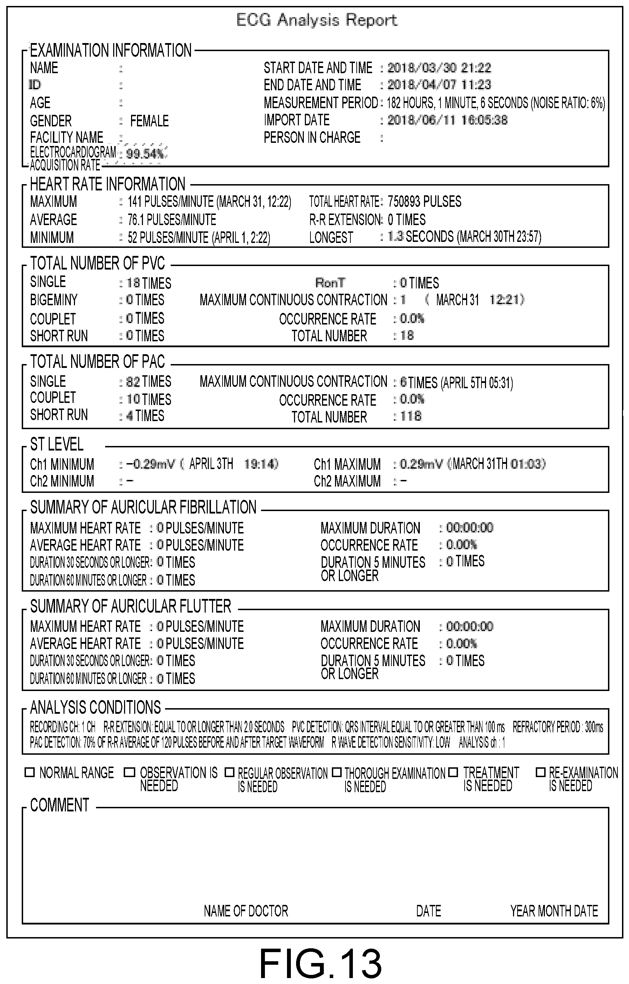

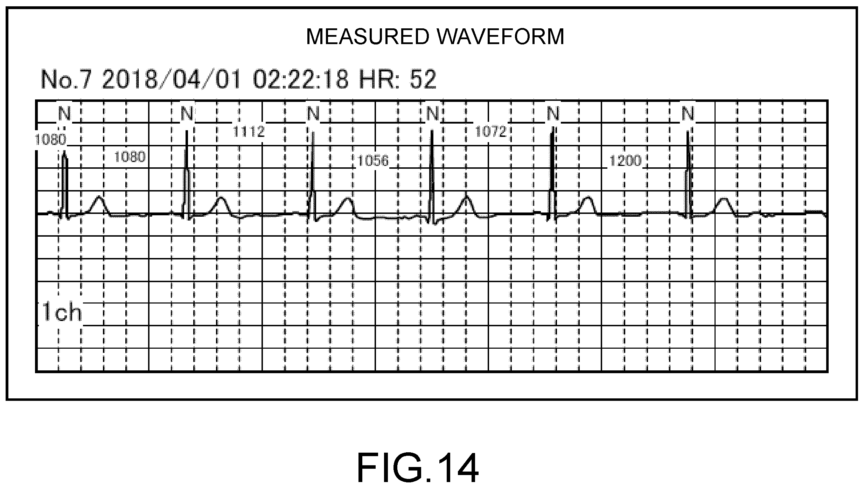

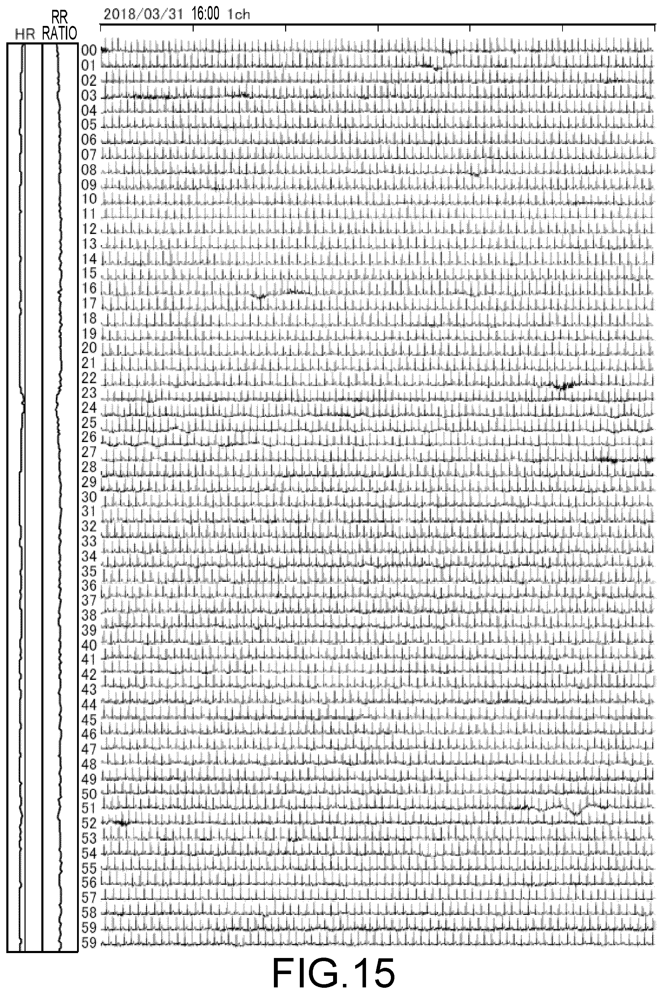

FIG. 13 to FIG. 15 illustrate part of the analysis report. FIG. 13 illustrates a front cover portion of the analysis report in which overview of the analysis result is summarized. Measurement results of heart rate information, premature ventricular contraction (PVC), premature atrial contraction (PAC), an ST level, auricular fibrillation and auricular flutter are summarized in one page. A stable electrocardiogram which can be analyzed is obtained during a long period. FIG. 14 illustrates one of measured waveforms recorded in the analysis report. FIG. 15 illustrates compressed waveforms which indicate the electrocardiogram of one hour summarized on one page.

The staff operates the analysis apparatus 500 to transmit data of the analysis report to the information processing apparatus 100. The data of the analysis report may be transmitted by email, may be transmitted using a file exchange system or may be uploaded to a dedicated web server of the medical institution. The data of the analysis report to be transmitted may be encrypted.

The doctor displays the received analysis report on a screen and diagnoses a state of the subject on the basis of the electrocardiogram and the analysis result. The doctor enters the result of the diagnosis in, for example, the electronic medical record. The doctor interviews the subject upon next visit to the hospital and tells the diagnosis result. In a case where the subject has a heart disease (abnormal cardiac rhythm) as a result of the diagnosis, the doctor discusses and determines courses of treatment in the future with the subject.

While the data of the analysis report is transmitted to the information processing apparatus 100 via the communication network 700 here, a recording medium including the data of the analysis report may be sent to the medical institution by mail, by courier, or the like. Alternatively, the data of the analysis report may be transmitted by fax. The analysis report may be provided using other methods.

Further, the electrocardiographic analysis institution may transmit the data of the analysis report to the medical institution via the medical equipment sales agency. In this case, the communication device 304 of the data processing apparatus 300 receives the data of the analysis report from the analysis apparatus 500 and stores the received data in the storage 303. The staff operates the data processing apparatus 300 to transmit the data of the analysis report to the information processing apparatus 100. The communication device 104 of the information processing apparatus 100 receives the data of the analysis report transmitted from the data processing apparatus 300 and stores the received data in the storage 103. The doctor displays the received analysis report and diagnoses a state of the subject on the basis of the electrocardiogram and the analysis result.

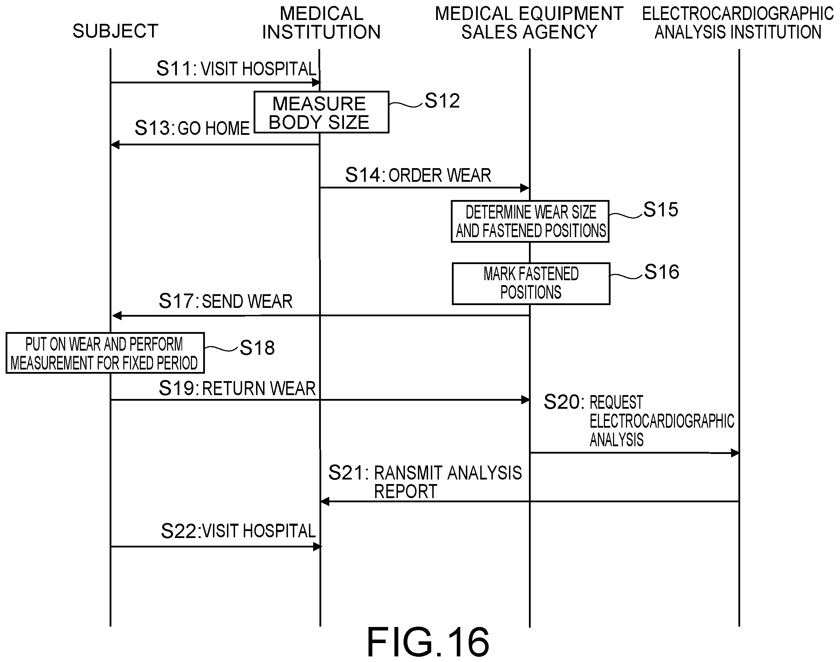

FIG. 16 is a sequence diagram of a specific example of the whole operation regarding the examination support system according to the present embodiment.

The subject visits the medical institution (S11). The doctor of the medical institution diagnoses the subject and enters the diagnosis result in the medical record. The medical record may be an electronic medical record or a paper medical record. The doctor judges that the subject is suspected to have a heart disease such as abnormal cardiac rhythm, determines to perform an electrocardiogram examination (Holter electrocardiogram examination) and issues a request form for Holter electrocardiogram examination. The request form may be issued using, for example, a request form print function provided at the electronic medical record. The doctor or the health personnel to whom the doctor provides directions gives an examination reception file including the request form to the subject. The subject presents the examination reception file to an examination room.

The laboratory technician measures the body size (such as under bust) of the subject and obtains size information including the under bust size (S12). Further, the laboratory technician explains how to use the wear which the subject puts on in the electrocardiogram examination and a measurement period. Then, the subject goes home (S13).

The doctor, the laboratory technician or other health personnel orders an electrocardiogram measurement set including the wear from the order screen using the information processing apparatus 100. Specifically, the doctor, the laboratory technician or other health personnel inputs the size of the under bust, name, an ID, shipment address, or the like, of the subject on the order screen. The information processing apparatus 100 transmits the order information on the electrocardiogram measurement set to the order reception apparatus 200 of the medical equipment sales agency (S14).

The order reception apparatus 200 receives the order information. The staff of the medical equipment sales agency activates the wear selection application of the order reception apparatus 200, inputs the size information (including at least the under bust) of the subject in the wear selection application and causes the wear selection application to calculate the wear size and the fastened positions (S15). The staff picks up the wear of the calculated size from the warehouse and marks the fastened positions (S16). In a case where the marks are provided on the wear in advance, the staff only requires to pick up the wear on which the fastened positions are marked from the warehouse and does not have to do marking work. The staff sends the electrocardiogram measurement set including the wear on which the marks are provided, the instruction manual, and the like, to the subject (S17). Note that it is also possible to send the wear to the subject in a state where the fastened positions are not marked. In this case, the electrocardiogram measurement set may include a notification which notifies the fastened positions to the subject. Alternatively, data of the fastened positions may be transmitted from the order reception apparatus 200, the inventory management apparatus 400, or the like, to the communication terminal 600 via the communication network 700.

The subject receives the electrocardiogram measurement set sent from the medical equipment sales agency. The subject puts on the wear in accordance with the instruction manual included in the electrocardiogram measurement set and explanation as to how to use the wear received from the health personnel (such as the doctor and the laboratory technician) in advance and records an electrocardiogram during the measurement period (S18). Specifically, the subject puts on the wear on the naked upper body and aligns the tips of the side tabs with the marked positions to thereby fix the wear on the body. The subject puts off the wear after the measurement period ends and returns the wear to the medical equipment sales agency (S19). It is also possible to return only the cardiography equipment loaded on the wear without returning the wear body portion or return the wear body portion at a later date.

The medical equipment sales agency receives the wear returned from the subject and causes the reader 305 of the data processing apparatus 300 to read out the measurement data (electrocardiographic data) stored in the cardiography equipment of the wear. The data processing apparatus 300 transmits analysis request information including the readout measurement data (electrocardiographic file) to the analysis apparatus 500 of the electrocardiographic analysis institution (S20).

The information processing apparatus 100 receives data of the analysis report including the analysis result from the analysis apparatus 500 (S21). The doctor of the medical institution diagnoses a state of the subject on the basis of the analysis result described in the analysis report. The doctor enters the diagnosis result in, for example, the electronic medical record. The doctor interviews the subject who visits the hospital (S22) and tells the diagnosis result. In a case where it is recognized as a result of the diagnosis that the subject has a heart disease (abnormal cardiac rhythm), the doctor discusses and determines courses of treatment in the future with the subject.

According to the present embodiment, the subject only requires to put on the wear sent from the medical equipment sales agency, record an electrocardiogram and return the wear which the subject has put on to the medical equipment sales agency after measurement, so that it is possible to reduce the number of times the subject visits the medical institution. In related art, for example, the subject needs to visit the hospital at least twice in addition to the first diagnosis and the diagnosis after the examination so as to have electrodes loaded on measurement positions of the body and have the electrodes removed after 24 hours. In contrast, in the present embodiment, the subject only requires to put on the sent wear and return the wear which the subject has put on and does not have to visit the hospital for loading and removal of electrodes, so that it is possible to reduce the number of times of visiting the hospital. It is therefore possible to reduce load on the subject.

Further, according to the present embodiment, the wear of the size appropriate for the subject is provided, so that it is possible to stably dispose the electrodes at correct positions and perform measurement comfortably and easily while allowing the subject himself/herself to easily put on and off the wear. Further, the positions where the tips of the side tabs are to be fastened are marked on the wear, so that the subject can fix the electrodes on the body with an appropriate pressure by fixing the tips of the side tabs at the marked positions. It is easy to always stably hold the electrode positions and the pressure, so that it is possible to record an electrocardiogram for a long period beyond 24 hours comfortably and with high accuracy.

Further, according to the present embodiment, it is possible to save work for loading of the electrodes on the subject and technical guidance by the medical institution which has been required in the Holter electrocardiogram examination for 24 hours in related art. It is therefore possible to reduce load on the medical institution. Further, the medical equipment is owned by the medical equipment sales agency and does not have to be owned by the medical institution, so that it is possible to reduce load on the medical institution also in this viewpoint.

Further, according to the present embodiment, the wear body portion can be cleaned and reused, so that it is possible to reduce the number of wears in stock and expect reduction in overall medical expenses.

### (Modified example 1)

In the above-described embodiment, the subject returns the wear which the subject has put on to the medical equipment sales agency and the medical equipment sales agency reads out measurement data from the wear. In the present modified example 1, the measurement data (electrocardiographic data) stored in a storage (such as a memory apparatus) inside the measurement equipment is transferred to the communication terminal 600 on the basis of operation by the subject, and the transferred measurement data is transmitted from the communication terminal 600 to the data processing apparatus 300. This enables the medical equipment sales agency to collect the measurement data early, so that the medical equipment sales agency can make an analysis request to the electrocardiographic analysis institution early. This enables the doctor to obtain the analysis report earlier.

### (Modified example 2)

While in the above-described embodiment, the medical institution (information processing apparatus 100) transmits the order information to the medical equipment sales agency, the subject may transmit the order information using the communication terminal 600. In this case, the communication terminal 600 is equipped with application for creating and transmitting the order information, and the subject inputs size information such as the under bust size to the application.

### (Modified example 3)

While in the above-described embodiment, the wear selector 205 of the order reception apparatus 200 calculates the wear size and the fastened positions, in a case where the medical institution keeps wears for trial fitting, at least one of the wear size and the fastened positions may be determined at the medical institution in step S12. In this case, a function for allowing input of at least one of the wear size and the fastened positions to the order input screen is provided at the order generator 105. The order generator 105 of the information processing apparatus 100 then calculates at least one of the wear size and the fastened positions from the under bust size, or the like. The information processing apparatus 100 transmits order information including the determined wear size and fastened positions to the order reception apparatus 200.

### (Modified example 4)

While in the above-described embodiment, the medical equipment sales agency sends the electrocardiogram measurement set to the subject, in a case where the medical institution also plays a role as a wear sales agency, the medical institution may send the electrocardiogram measurement set. In this case, the medical institution determines the wear size and the fastened positions in accordance with modified example 3. Further, the subject may return the wear to the medical institution. Further, the medical institution may read out measurement data from the wear and may make an analysis request to the electrocardiographic analysis institution.

### (Modified example 5)

In the above-described embodiment, a length adjustment mechanism may be provided at two shoulder belts 33 (see FIG. 3) or the torso portion 34 (see FIG. 5) so that the length of the two shoulder belts 33 or the torso portion 34 can be adjusted. The length adjustment mechanism may have any structure. For example, part of the shoulder belt may be formed in a circular shape and the length can be adjusted by changing a size of the circular portion. By adjusting the length in this manner, it is possible to prepare wears of a plurality of sizes such as S, M and L using wears which originally have the same size. Candidates for adjustment positions of the shoulder belt may be expressed with scales on the shoulder belt in a similar manner to the fastened positions of the side tabs. The wear may have a form without shoulder belts. Further, the order reception apparatus 200 or the information processing apparatus 100 may calculate the adjustment positions from the size information on the subject and provide a wear on which the determined positions are marked to the subject. It is only necessary to obtain and create correspondence relationship between the adjustment positions and the size information in advance from a number of samples.

### Reference Signs List

1 measurement wear (wear)
10 cardiography equipment (measurement equipment)
20, 21, 22 electrode
30 wear body portion
31 front body
32 rear body
33 shoulder belt
34 torso portion
35 elastic body
36 side tab
37 connector
40 hook and loop fastener B side
41 stitch
100 information processing apparatus
200 order reception apparatus
300 data processing apparatus
400 inventory management apparatus
500 analysis apparatus
600 communication terminal
700 communication network
101, 201, 301 input device
102, 202, 302 display
103, 203, 303 storage
104, 204, 304 communication device
105 order generator
205 wear selector
206 shipment instruction device
305 reader
306 analysis request device
401 warehouse

## Claims

1. An examination support method for supporting an electrocardiogram examination of a subject using a wear on which medical equipment including electrocardiography equipment, a plurality of electrodes and one or more size adjustment mechanisms are loaded, the examination support method comprising the consecutive steps of:
measuring (S12) body size information including an underbust size of the subject using a first measurement apparatus;
ordering (S14) a wear using an information processing apparatus (100), the ordering including transmitting order instruction information including the body size information to an order reception apparatus (200);
selecting a wear to be put on the subject from a plurality of wears of a plurality of sizes on a basis of the underbust size of the subject received by the order reception apparatus;
determining (205, 211), by the order reception apparatus (200), an adjustment position (RP1-21, PM1-21, PJ-21) of the one or more size adjustment mechanisms on the wear based on the underbust size;
sending (S16, S17) the selected wear on which the adjustment position is indicated to the subject;
putting (S18) the wear on a torso portion of the subject;
adjusting a pressure on the torso portion of the subject by positioning the one or more size adjustment mechanisms at the adjustment position;
performing electrocardiogram measurements for a period during which the subject is wearing the wear;
returning the wear (S19);
connecting a data processing apparatus wiredly or wirelessly with the medical equipment loaded on the returned wear and reading out electrocardiogram measurement data measured for the period during which the subject has put on the wear;
reguesting (S20) electrocardiographic analysis by transmitting the electrocardiogram measurement data to an analysis apparatus (500) for electrocardiographic analysis; and
performing (S21, S22) the electrocardiogram examination by the analysis apparatus.

2. The examination support method according to claim 1,
wherein the body size information further includes at least one of a body height, a chest measurement, a shoulder width, a sitting height, and a body weight.

3. The examination support method according to claim 1,
wherein in the selecting, a wear with a mark indicating the determined adjustment position with which the size adjustment mechanisms are to be aligned is selected among wears having the determined size.

4. The examination support method according to any one of claims 1 to 3,
wherein the wear has scales indicating candidates for the adjustment position with which the size adjustment mechanisms are to be aligned.

5. The examination support method according to claim 4,
wherein the number of the size adjustment mechanisms is one, and an interval of the scales is equal to or greater than 2 cm and equal to or less than 16 cm, or the number of the size adjustment mechanisms is two, and an interval of the scales is equal to or greater than 1 cm and equal to or less than 8 cm.

6. The examination support method according to claim 1, further comprising:
transmitting information indicating the determined adjustment position to a communication terminal of the subject.

7. The examination support method according to any one of claims 1 to 6, further comprising:
acquiring analysis result data by analyzing the electrocardiogram measurement data read out by the data processing apparatus.

8. The examination support method according to any one of claims 1 to 7,
wherein the wear includes a shoulder belt.

9. The examination support method according to any one of claims 1 to 8,
wherein the electrocardiography equipment is able to operate for equal to or longer than 48 hours in a state where the electrocardiography equipment is fully charged.

10. The examination support method according to any one of claims 1 to 9,
wherein the electrodes and the cardiography equipment are detachable from the wear.

11. The examination support method according to any one of claims 1 to 10,
wherein the electrocardiography equipment includes a display configured to display an operation state of the electrocardiography equipment, and
the examination support method comprises:
displaying the operation state of the electrocardiography equipment at the display.

12. An examination support system for supporting an electrocardiogram examination of a subject using a wear (1) on which medical equipment including electrocardiography equipment, a plurality of electrodes (20, 21, 22) and one or more size adjustment mechanisms (36, 40) are loaded, the examination support system comprising:
a first measurement apparatus configured to measure body size information including an underbust size of the subject;
an information processing apparatus (100) which comprises:
an input device (101) configured to input the body size information into an ordering application for ordering wear; and
a first communication device (104) configured to transmit order instruction information comprising the body size information;
an order reception apparatus (200) which comprises:
a second communication device (204) configured to receive the order instruction information from the information processing apparatus; and
a wear selector (205) configured to select a wear to be put on the subject from a plurality of wears of a plurality of sizes in accordance with the order instruction information, based on the underbust size; wherein the wear selector is further configured to determine an adjustment position (Ps1-21, Pm1-21, PI1-21) of the one or more size adjustment mechanisms on the wear based on the underbust size;
means for sending the selected wear on which the adjustment position is indicated to the subject;
means for returning the wear after the subject has put on the wear for a predetermined period during which electrocardiogram measurements are performed;
a data processing apparatus (300) which comprises:
a reader (305) configured to connect wiredly or wirelessly with the medical equipment loaded on the returned wear and read out electrocardiogram measurement data measured during the period in which the subject has put on the wear;
an analysis apparatus (500) configured to receive a reguest for electrocardiographic analysis and the corresponding electrocardiogram measurement data from the data processing apparatus, and perform an electrocardiogram examination,
wherein the one or more size adjustment mechanisms are configured for adjusting a pressure on a torso portion of the subject by positioning the one or more size adjustment mechanisms at the adjustment position; and
wherein the wear has scales (41) indicating candidates for the adjustment position with which the size adjustment mechanisms are to be aligned.

## Patentansprüche

1. Ein Untersuchungsunterstützungsverfahren zum Unterstützen einer Elektrokardiogramm-Untersuchung einer Person unter Verwendung eines Kleidungsstücks, an dem eine medizinische Ausstattung einschließlich einer Elektrokardiographie-Ausstattung, eine Vielzahl von Elektroden und ein oder mehrere Größeneinstellungsmechanismen angebracht sind, wobei das Untersuchungsunterstützungsverfahren die folgenden aufeinanderfolgenden Schritte umfasst:
Messen (S12) von Körpergrößeninformationen einschließlich einer Unterbrustgröße der Person unter Verwendung einer ersten Messvorrichtung,
Bestellen (S14) eines Kleidungsstücks unter Verwendung einer Informationsverarbeitungsvorrichtung (100), wobei das Bestellen das Senden von Bestellanweisungsinformationen einschließlich der Körpergrößeninformationen an eine Bestellungsempfangsvorrichtung (200) umfasst,
Auswählen eines Kleidungsstücks für das Tragen durch die Person aus einer Vielzahl von Kleidungsstücken mit einer Vielzahl von Größen basierend auf der durch die Bestellungsempfangsvorrichtung empfangenen Unterbrustgröße der Person,
Bestimmen (205, 211), durch die Bestellungsempfangsvorrichtung (200), einer Einstellungsposition (RP1-21, PM1-21, PJ-21) des einen oder der mehreren Größeneinstellungsmechanismen an dem Kleidungsstück basierend auf der Unterbrustgröße,
Versenden (S16, S17) des ausgewählten Kleidungsstücks, an dem die Einstellungsposition angegeben ist, an die Person,
Anlegen (S18) des Kleidungsstücks an einem Torsoteil der Person,
Einstellen eines Drucks auf den Torsoteil der Person durch das Positionieren des einen oder der mehreren Größeneinstellungsmechanismen an der Einstellungsposition,
Durchführen von Elektrokardiogrammmessungen für eine Zeitdauer, während welcher die Person das Kleidungsstück trägt,
Zurücksenden des Kleidungsstücks (S19),
Verbinden einer Datenverarbeitungsvorrichtung drahtgebunden oder drahtlos mit der an dem zurückgesendeten Kleidungsstück angebrachten medizinischen Ausstattung und Auslesen von Elektrokardiogramm-Messdaten, die für die Zeitdauer, während welcher die Person das Kleidungsstück getragen hat, gemessen wurden,
Anfordern (S20) einer elektrokardiographischen Analyse durch das Senden der Elektrokardiogramm-Messdaten an eine Analysevorrichtung (500) für eine elektrokardiographische Analyse, und
Durchführen (S21, S22) der Elektrokardiogramm-Untersuchung durch die Analysevorrichtung.

2. Untersuchungsunterstützungsverfahren nach Anspruch 1,
wobei die Körpergrößeninformationen weiterhin eine Körperlänge, ein Brustmaß, eine Schulterbreite, eine Sitzhöhe und/oder ein Körpergewicht umfassen.

3. Untersuchungsunterstützungsverfahren nach Anspruch 1,
wobei bei dem Auswählen ein Kleidungsstück mit einer Markierung, die die bestimmte Einstellungsposition, mit der die Größeneinstellungsmechanismen auszurichten sind, angibt, innerhalb von Kleidungsstücken mit der bestimmten Größe ausgewählt wird.

4. Untersuchungsunterstützungsverfahren nach einem der Ansprüche 1 bis 3,
wobei das Kleidungsstück Skalen aufweist, die Kandidaten für die Einstellungsposition, mit der die Größeneinstellungsmechanismen auszurichten sind, angeben.

5. Untersuchungsunterstützungsverfahren nach Anspruch 4,
wobei die Anzahl der Größeneinstellungsmechanismen gleich eins ist und ein Intervall der Skalen gleich oder größer als 2 cm und gleich oder kleiner als 16 cm ist, oder wobei die Anzahl der Größeneinstellungsmechanismen gleich zwei ist und ein Intervall der Skalen gleich oder größer als 1 cm und gleich oder kleiner als 8 cm ist.

6. Untersuchungsunterstützungsverfahren nach Anspruch 1, das weiterhin umfasst:
Senden von Informationen, die die bestimmte Einstellungsposition angeben, an ein Kommunikationsendgerät der Person.

7. Untersuchungsunterstützungsverfahren nach einem der Ansprüche 1 bis 6, das weiterhin umfasst:
Erhalten von Analyseergebnisdaten durch das Analysieren der durch die Datenverarbeitungsvorrichtung ausgelesenen Elektrokardiogramm-Messdaten.

8. Untersuchungsunterstützungsverfahren nach einem der Ansprüche 1 bis 7,
wobei das Kleidungsstück einen Schultergürtel umfasst.

9. Untersuchungsunterstützungsverfahren nach einem der Ansprüche 1 bis 8,
wobei die Elektrokardiographie-Ausstattung befähigt ist für einen Betrieb gleich oder länger als 48 Stunden in einem Zustand, in dem die Elektrokardiographie-Ausstattung vollständig geladen ist.

10. Untersuchungsunterstützungsverfahren nach einem der Ansprüche 1 bis 9,
wobei die Elektroden und die Elektrokardiographie-Ausstattung von dem Kleidungsstück entfernt werden können.

11. Untersuchungsunterstützungsverfahren nach einem der Ansprüche 1 bis 10,
wobei die Elektrokardiographie-Ausstattung ein Display umfasst, das konfiguriert ist zum Anzeigen eines Betriebszustands der Elektrokardiographie-Ausstattung, und
das Untersuchungsunterstützungsverfahren umfasst:
Anzeigen des Betriebszustands der Elektrokardiographie-Ausstattung an dem Display.

12. Untersuchungsunterstützungssystem für das Unterstützen einer Elektrokardiogramm-Untersuchung einer Person unter Verwendung eines Kleidungsstücks (1), an dem eine medizinische Ausstattung einschließlich einer Elektrokardiographie-Ausstattung, eine Vielzahl von Elektroden (20, 21, 22) und ein oder mehrere Größeneinstellungsmechanismen (36, 40) angebracht sind, wobei das Untersuchungsunterstützungssystem umfasst:
eine erste Messvorrichtung, die konfiguriert ist zum Messen von Körpergrößeninformationen einschließlich einer Unterbrustgröße der Person,
eine Informationsverarbeitungsvorrichtung (100), die umfasst:
eine Eingabevorrichtung (101), die konfiguriert ist zum Eingeben der Körpergrößeninformationen in eine Bestellanwendung für das Bestellen eines Kleidungsstücks, und
eine erste Kommunikationsvorrichtung (104), die konfiguriert ist zum Senden von Bestellanweisungsinformationen, die die Körpergrößeninformationen umfassen,
eine Bestellungsempfangsvorrichtung (200), die umfasst:
eine zweite Kommunikationsvorrichtung (204), die konfiguriert ist zum Empfangen der Bestellungsanweisungsinformationen von der Informationsverarbeitungsvorrichtung, und
einen Kleidungsstück-Auswähler (205), der konfiguriert ist zum Auswählen eines durch die Person zu tragenden Kleidungsstücks aus einer Vielzahl von Kleidungsstücken mit einer Vielzahl von Größen gemäß den Bestellungsanweisungsinformationen basierend auf der Unterbrustgröße, wobei der Kleidungsstück-Auswähler weiterhin konfiguriert ist zum Bestimmen einer Einstellungsposition (Ps1-21, Pm1-21, PI1-21) des einen oder der mehreren Größeneinstellungsmechanismen an dem Kleidungsstück basierend auf der Unterbrustgröße,
Mittel zum Versenden des ausgewählten Kleidungsstücks, an dem die Einstellungsposition für die Person angegeben ist,
Mittel zum Zurücksenden des Kleidungsstücks, nachdem die Person das Kleidungsstück für eine vorbestimmte Zeitdauer, während welcher Elektrokardiogramm-Messungen durchgeführt wurden, getragen hat,
eine Datenverarbeitungsvorrichtung (300), die umfasst:
einen Leser (305), der konfiguriert ist zum drahtgebundenen oder drahtlosen Verbinden mit der an dem zurückgesendeten Kleidungsstück angebrachten medizinischen Ausstattung und zum Auslesen von Elektrokardiogramm-Messdaten, die während der Zeitdauer, während welcher die Person das Kleidungsstück getragen hat, gemessen wurden,
eine Analysevorrichtung (500), die konfiguriert ist zum Empfangen einer Aufforderung für eine elektrokardiographische Analyse und der entsprechenden Elektrokardiogramm-Messdaten von der Datenverarbeitungsvorrichtung und zum Durchführen einer Elektrokardiogramm-Untersuchung,
wobei der eine oder die mehreren Größeneinstellungsmechanismen konfiguriert sind zum Einstellen eines Drucks auf einen Torsoteil der Person durch das Positionieren des einen oder der mehreren Größeneinstellungsmechanismen an der Einstellungsposition, und
wobei das Kleidungsstück Skalen (41) aufweist, die Kandidaten für die Einstellungsposition, mit der die Größeneinstellungsmechanismen auszurichten sind, angeben.

## Revendications

1. Procédé d'assistance à un examen pour assister un examen électrocardiographique d'un sujet à l'aide d'un dispositif portable, sur lequel sont chargés un équipement médical comprenant un équipement d'électrocardiographie, une pluralité d'électrodes et un ou plusieurs mécanismes de réglage de taille, le procédé d'assistance à un examen comprenant les étapes consécutives suivantes :
la mesure (S12) d'informations de taille corporelle, y compris un tour de poitrine du sujet, à l'aide d'un premier appareil de mesure ;
la commande (S14) d'un dispositif portable à l'aide d'un dispositif de traitement d'informations (100),
la commande comprenant la transmission d'informations d'instruction de commande comprenant les informations de taille corporelle à un appareil de réception de commande (200) ;
la sélection d'un dispositif portable à placer sur le sujet parmi une pluralité de dispositifs portables d'une pluralité de tailles, sur la base du tour de poitrine du sujet reçu par l'appareil de réception de commande ;
la détermination (205, 211), par l'appareil de réception de commande (200), d'une position de réglage (R.P1-21, PM1-21, PJ-21) des un ou plusieurs mécanismes de réglage de taille sur le dispositif portable en fonction du tour de poitrine ;
l'envoi (S16, S17) du dispositif portable sélectionné sur lequel la position de réglage est indiquée au sujet ;
la mise en place (S18) du dispositif portable sur une partie du torse du sujet ; le réglage d'une pression sur la partie du torse du sujet en positionnant les un ou plusieurs mécanismes de réglage de taille à la position de réglage ;
l'exécution de mesures électrocardiographiques pendant une période au cours de laquelle le sujet porte le dispositif portable ;
le retour du dispositif portable (S19) ;
la connexion d'un appareil de traitement de données, par fil ou sans fil, à l'équipement médical chargé sur le dispositif portable retourné et la lecture des données de mesure d'électrocardiogramme mesurées pendant la période pendant laquelle le sujet a porté le dispositif portable ;
la demande (S20) d'analyse électrocardiographique en transmettant les données de mesure d'électrocardiogramme à un appareil d'analyse (500) pour analyse électrocardiographique ; et
l'exécution (S21, S22) de l'examen électrocardiographique par l'appareil d'analyse.

2. Procédé d'assistance à un examen selon la revendication 1, dans lequel
les informations de taille corporelle comprennent en outre au moins un élément parmi une taille corporelle, une mesure de poitrine, une largeur d'épaules, une hauteur assise et un poids corporel.

3. Procédé d'assistance à un examen selon la revendication 1,
dans lequel, lors de la sélection, un dispositif portable présentant une marque indiquant la position de réglage déterminée avec laquelle les mécanismes de réglage de taille doivent être alignés est sélectionnée parmi les dispositifs portables ayant la taille déterminée.

4. Procédé d'assistance à un examen selon l'une quelconque des revendications 1 à 3,
dans lequel le dispositif portable présente des échelles indiquant des candidates pour la position de réglage avec laquelle les mécanismes de réglage de taille doivent être alignés.

5. Procédé d'assistance à un examen selon la revendication 4,
dans lequel le nombre de mécanismes de réglage de taille est égal à un, et un intervalle des échelles est supérieur ou égal à 2 cm et inférieur ou égal à 16 cm, ou le nombre de mécanismes de réglage de la taille est égal à deux, et l'intervalle entre les échelles est supérieur ou égal à 1 cm et inférieur ou égal à 8 cm.

6. Procédé d'assistance à un examen selon la revendication 1, comprenant en outre :
la transmission d'informations indiquant la position de réglage déterminée à un terminal de communication du sujet.

7. Procédé d'assistance à un examen selon l'une quelconque des revendications 1 à 6, comprenant en outre :
l'acquisition de données de résultats d'analyse en analysant les données de mesure d'électrocardiogramme lues par l'appareil de traitement de données.

8. Procédé d'assistance à un examen selon l'une quelconque des revendications 1 à 7,
dans lequel le dispositif portable comprend une sangle d'épaule.

9. Procédé d'assistance à un examen selon l'une quelconque des revendications 1 à 8,
dans lequel l'équipement d'électrocardiographie est capable de fonctionner pendant une durée égale ou supérieure à 48 heures dans un état où l'équipement d'électrocardiographie est complètement chargé.

10. Procédé d'assistance à un examen selon l'une quelconque des revendications 1 à 9,
dans lequel les électrodes et l'équipement de cardiographie sont détachables du dispositif portable.

11. Procédé d'assistance à un examen selon l'une quelconque des revendications 1 à 10,
dans lequel l'équipement d'électrocardiographie comprend un afficheur configuré pour afficher un état de fonctionnement de l'équipement d'électrocardiographie, et
le procédé d'assistance à un examen comprend :
l'affichage de l'état de fonctionnement de l'équipement d'électrocardiographie sur l'afficheur.

12. Système d'assistance à un examen pour assister à un examen électrocardiographique d'un sujet à l'aide d'un dispositif portable (1) sur lequel sont chargés un équipement médical comprenant un équipement d'électrocardiographie, une pluralité d'électrodes (20, 21, 22) et un ou plusieurs mécanismes de réglage de taille (36, 40), le système d'assistance à l'examen comprenant :
un premier appareil de mesure configuré pour mesurer des informations de taille corporelle, y compris le tour de poitrine du sujet ;
un appareil de traitement d'informations (100) qui comprend :
un dispositif de saisie (101) configuré pour saisir les informations de taille corporelle dans une application de commande pour commander un dispositif portable ; et
un premier dispositif de communication (104) configuré pour transmettre des informations d'instructions de commande comprenant les informations de taille corporelle ;
un appareil de réception de commande (200) qui comprend :
un deuxième dispositif de communication (204) configuré pour recevoir les informations d'instruction de commande provenant de l'appareil de traitement d'informations ; et
un sélecteur de dispositif portable (205) configuré pour sélectionner un dispositif portable à placer sur le sujet parmi une pluralité de dispositifs portables d'une pluralité de tailles conformément aux informations d'instruction de commande, sur la base du tour poitrine ; dans lequel le sélecteur de dispositif portable est en outre configuré pour déterminer une position de réglage (Ps1-21, Pm1-21, PI1-21) des un ou plusieurs mécanismes de réglage de taille sur le dispositif portable sur la base du tour de poitrine ;
des moyens pour envoyer au sujet le dispositif portable sélectionné sur lequel la position de réglage est indiquée ;
des moyens pour retourner le dispositif portable après que le sujet a porté le dispositif portable pendant une période prédéterminée au cours de laquelle des mesures électrocardiographiques sont effectuées ;
un appareil de traitement de données (300) qui comprend :
un lecteur (305) configuré pour se connecter par fil ou sans fil avec l'équipement médical chargé sur le dispositif portable retourné et lire des données de mesure d'électrocardiogramme mesurées pendant la période où le sujet a porté le dispositif portable ;
un appareil d'analyse (500) configuré pour recevoir une demande d'analyse électrocardiographique et les données de mesure d'électrocardiogramme correspondantes provenant de l'appareil de traitement de données, et pour effectuer un examen électrocardiographique,
dans lequel les un ou plusieurs mécanismes de réglage de taille sont configurés pour régler une pression sur une partie du torse du sujet en positionnant les un ou plusieurs mécanismes de réglage de taille à la position de réglage ; et
dans lequel le dispositif portable présente des échelles (41) indiquant des candidates pour la position de réglage avec laquelle les mécanismes de réglage de taille doivent être alignés.
